# EUROPEAN PATENT APPLICATION

(11) **EP 4 653 453 A1**
(43) Date of publication of application: **26.11.2025**
(21) Application number: 23917005.3
(22) Date of filing: 12.07.2023
(51) Int. Cl.: C07K 14/065, A61K 39/275, A61P 31/12, C12N 15/39, C07K 19/00, C12N 15/62, A61P 31/20

(54) **RECOMBINANT CHIMERIC ANTIGEN FOR POXVIRUS, SUBUNIT VACCINE COMPRISING SAME AND USE THEREOF**

(30) Priority: 18.01.2023 CN 202310086433
(71) Applicant: Peking University, Beijing 100871 (CN); Institute Of Microbiology, Chinese Academy Of Sciences, Beijing 100101 (CN); SHANXI ACADEMY OF ADVANCED RESEARCH AND INNOVATION, Shanxi 030032 (CN); Beihang University, Beijing 100191 (CN)
(72) Inventor: XI, Jianzhong Jeff, Beijing 100871 (CN); GAO, George Fu, Beijing 100871 (CN); WANG, Han, Beijing 100871 (CN); YIN, Peng, Beijing 100871 (CN); LI, Shihua, Beijing 100871 (CN); QU, Xiao, Beijing 100871 (CN); WANG, Qihui, Beijing 100871 (CN); LIU, Zhida, Beijing 100871 (CN); CUI, Qingwei, Beijing 100871 (CN)
(74) Representative: Plougmann Vingtoft a/s
(86) International application number: PCT/CN2023/107061
(87) International publication number: WO 2024/152533

(57) **Abstract**

Provided are a recombinant chimeric antigen for a poxvirus, in particular for a Mpoxvirus, a subunit vaccine comprising the recombinant chimeric antigen, and a use thereof. The recombinant chimeric antigen of the present application comprises two immunogens arranged in a specific manner: a Mpoxvirus A35 protein or an antigenic fragment thereof or derivative peptides of same, and a Mpoxvirus M1 protein or an antigenic fragment thereof or derivative peptides of same, and can excite an immune response to two infectious virus particles of intracellular mature virus (IMV) particles and extracellular enveloped virus (EEV) particles.

## Description

### Cross Reference

This application claims priority to the Chinese patent application filed on January 18, 2023 with application number 202310086433.2 and the invention title "Recombinant Chimeric Antigen for Poxvirus, Subunit Vaccine Comprising the Same and Use thereof", the entire content of which is incorporated herein by reference.

### Technical Field

This application belongs to the field of biomedicine, and specifically relates to a recombinant chimeric antigen for poxvirus, subunit vaccine comprising the same and use thereof.

### Background

Poxviruses, represented by monkeypox virus, are a type of nucleocytoplasmic large DNA virus with a viral genome of approximately 130-375 kbp, which can encode up to 200 viral proteins. In addition to encoding the viral proteins, the virus particles of poxviruses are also relatively complex, which have two morphologically different infectious viral particles, called intracellular mature virus (IMV) and extracellular enveloped virus (EEV). Among them, IMV has an envelope, which is more stable than EEV and is mainly involved in the transmission of the virus between hosts. EEV has a special outer membrane structure, which is mainly involved in the spread of the virus in the host body. Since IMV and EEV have different membrane structures, membrane components, and cell infection mechanisms, their surface neutralizing antigens are also completely different.

Monkeypox is a viral zoonosis caused by infection with monkeypox virus (Mpoxvirus, MPXV). Monkeypox virus and Variola virus both belong to the same genus, Orthopoxvirus of the Poxviridae family. This genus includes four human pathogenic viruses, namely Variola virus (VARV), Monkeypox virus, and Cowpox virus (CPXV) and Vaccinia virus (VACV). Monkeypox virus was first isolated and discovered in 1958 in laboratory monkeys by Danish scientists, and the first human infection case was discovered in 1970 in the Congo (DRC). After that, monkeypox virus spread locally in central and western Africa for a long time, and evolved into two branches: West Africa and the Congo Basin (Central Africa). The Central African branch has strong transmission ability and pathogenicity, with a mortality rate of about 10.6%, and it is mainly prevalent in African countries such as Gabon, Cameroon, Congo (DRC), Congo (Brazzaville) and Sudan; the West African branch has weaker transmission ability and pathogenicity, with a mortality rate of about 3.6%, and it mainly appears in West African countries such as Nigeria, Liberia, Ghana and Sierra Leone, and countries outside Africa such as Europe. Since the first case of monkeypox was confirmed in the United Kingdom in May 2022, confirmed cases of monkeypox epidemic have appeared in many countries including the United States, Italy, Sweden, Spain, Portugal, Belgium, Germany, Australia, and the monkeypox virus is showing a global epidemic trend. On July 23, 2022 local time, the World Health Organization (WHO) officially announced that the monkeypox epidemic constituted a "Public Health Emergency of International Concern" (PHEIC). This is another highest level alert issued by the WHO following the 2020 COVID-19 epidemic. As of October 7, 2022, a total of 71,237 cases of monkeypox have been reported worldwide, including 26 deaths, involving 107 countries and regions. In addition, although variola virus was eradicated in the 1980s, its pathogens still exist and could pose a renewed threat to human health.

At present, there is no vaccine specifically developed for monkeypox virus in the world. Only two vaccines can be used to protect against monkeypox virus infection, namely JYNNEOS^{™} vaccine (also known as Imvamune or Imvanex) produced by Ankara-Bavarian Nordic and ACAM2000^{®} produced by Sanofi Pasteur. Both vaccines are attenuated live vaccines originally used to prevent variola. Among them, ACAM2000^{®} is a second-generation vaccine that has the ability to replicate in the human body. After vaccination, there is a risk of encephalitis, myocarditis, progressive cowpox infection, etc., and it is not suitable for vaccination in young children, pregnant women and people with low or compromised immune function. JYNNEOS^{™} is a third-generation vaccine. Because it cannot replicate in the human body, it is safer than the first- and second-generation vaccines. However, its immune effect has also been reduced to a certain extent. It is worth noting that the above-mentioned vaccine products were approved after the eradication of the variola virus, Therefore, they have not been widely used for vaccination among the population. Their ability to curb the spread of the variola virus and the monkeypox virus and to eradicate these viruses remains to be determined.

In short, the currently approved vaccines are all attenuated live vaccines developed for variola virus, and their ability to curb the spread of monkeypox virus remains to be verified. In addition, attenuated live viruses have obvious side effects from vaccination and may face the risk and uncertainty of mutations that could cause the virus to become more virulent. These issues limit the population for whom the vaccine can be administered, making it unsuitable for young children, pregnant women, and individuals with low or compromised immune function.

In addition to the limited vaccination population and clear side effects of vaccination, there are also uncertainties and safety risks associated with attenuated live vaccines. First, there is the safety risk of potential mutations in vaccine strains that could lead to reversion to virulence. Second, as a nucleocytoplasmic large DNA virus, the poxvirus genome can encode up to 200 viral proteins. Therefore, the antigenic components carried by live virus vaccines are extremely complex, and the effective immunogens and mechanisms of action remain unclear. These include a variety of viral proteins with immunosuppressive functions, which can negatively impact both the vaccine's immunity efficacy and the host's immune system. These safety risks and uncertainties are particularly evident in individuals with weakened immune systems, such as the elderly and HIV carriers. This further limits the vaccination of such immune-vulnerable groups.

Not only that, the live virus nature of attenuated live vaccines leads to low production capacity and high cost, which cannot meet the needs of large-scale population vaccination and emergency vaccine production. Therefore, in view of the various shortcomings of existing vaccines and in response to the recent global epidemic trend of monkeypox virus, as well as the potential future threats posed by other poxviruses such as variola, it is urgently necessary to employ new technologies to develop a new generation of vaccines, which have clear immunogenic components, well-defined mechanisms of action, and ensure safety, effectiveness and rapid availability, to assist in disease prevention and control.

The information disclosed in this background section is merely intended to increase the understanding of the overall background of the application. It should not be regarded as an admission or any form of suggestion that the information constitutes the prior art already known to a person skilled in the art.

### Summary of the Invention

### Aim of the Invention

In view of the various shortcomings of the existing vaccines, the present application aims to provide a recombinant chimeric antigen for monkeypox virus that can efficiently elicit a specific immune response against monkeypox virus (for example, producing protective antibodies), its related vaccine products, preparation method and applications. Additionally, vaccine products based on the recombinant chimeric antigen also have the advantages of safety, efficacy, clear immunogenic composition and protection mechanism, high production capacity, low cost. thereby enabling the vaccine to meet the safety and production requirements of large-scale emergency vaccine campaigns.

### Solution

To achieve the aim of the present application, the following technical solutions are provided:
In a first aspect, the present application provides a recombinant chimeric antigen for poxvirus. The recombinant chimeric antigen comprising an amino acid sequence arranged according to the following formula (I):

A1-C₁-M1-C₂-A2-C₃-M2 (I)

In formula (I):
A1 represents the Monkeypox virus A35 protein (encoded by the monkeypox virus A35R gene) or antigenic fragment I thereof, or an amino acid sequence having at least 90%, 92%, 95%, 96%, 97%, 98% or 99% identity thereto and having the same or substantially the same immunogenicity thereto,
A2 represents the Monkeypox virus A35 protein (encoded by the monkeypox virus A35R gene) or antigenic fragment II thereof, or an amino acid sequence having at least 90%, 92%, 95%, 96%, 97%, 98% or 99% identity thereto and having the same or substantially the same immunogenicity thereto,
M1 represents the Monkeypox virus M1 (encoded by the monkeypox virus M1R gene) protein or antigenic fragment I thereof, or an amino acid sequence having at least 90%, 92%, 95%, 96%, 97%, 98% or 99% identity thereto and having the same or substantially the same immunogenicity thereto,
M2 represents the Monkeypox virus M1 (encoded by the monkeypox virus M1R gene) protein or antigenic fragment II thereof, or an amino acid sequence having at least 90%, 92%, 95%, 96%, 97%, 98% or 99% identity thereto and having the same or substantially the same immunogenicity thereto;
C1, C2, C3 are each independently absent, or a linking sequence (GGGGS)n, wherein n is any integer between 1 and 10;
wherein,
A1 and A2 may be the same or different,
M1 and M2 may be the same or different.

In a feasible implementation, the antigenic fragment I or II of the A35 protein is the extracellular segment of the protein or a part thereof; and/or the antigenic fragment I or II of the M1 protein is the extracellular segment of the protein or a part thereof.

In some embodiments:
A1 represents the amino acid sequence as shown in SEQ ID NO: 1, or an amino acid sequence having the same or substantially the same immunogenicity obtained by substituting, deleting or adding one or more amino acids to the amino acid sequence as shown in SEQ ID NO: 1;
and/or, A2 represents the amino acid sequence as shown in SEQ ID NO:1, or an amino acid sequence of the amino acid sequence as shown in SEQ ID NO:1 with a fragment of 1-30 amino acids extended to the N-terminus of the A35 protein, or an amino acid sequence having the same or substantially the same immunogenicity thereto obtained by substituting, deleting or adding one or more amino acids to the above amino acid sequences; preferably, A2 represents the amino acid sequence as shown in SEQ ID NO:2, or an amino acid sequence having the same or substantially the same immunogenicity thereto obtained by substituting, deleting or adding one or more amino acids to the amino acid sequence as shown in SEQ ID NO: 2;
and/or, M1 and M2 are the same and represent the amino acid sequence as shown in SEQ ID NO:3, or an amino acid sequence having the same or substantially the same immunogenicity thereto obtained by substituting, deleting or adding one or more amino acids to the amino acid sequence as shown in SEQ ID NO: 3.

In a preferred embodiment:
A1 represents the amino acid sequence as shown in SEQ ID NO: 1;
A2 represents the amino acid sequence shown in SEQ ID NO: 1 or SEQ ID NO: 2;
M1 and M2 are the same and represent the amino acid sequence as shown in SEQ ID NO:3.

Further preferably, A1 represents the amino acid sequence as shown in SEQ ID NO: 1;
A2 represents the amino acid sequence as shown in SEQ ID NO: 2;
M1 and M2 are the same and represent the amino acid sequence as shown in SEQ ID NO:3.

Optionally, C1, C2, and C3 are all absent.

Further preferably, the recombinant chimeric antigen comprises the amino acid sequence as shown in SEQ ID NO: 4.

In a specific embodiment, the two A35R proteins or the antigenic fragments thereof in the recombinant chimeric antigen can dimerize under appropriate conditions to form a stable single-chain dimer structure. Therefore, in a specific embodiment, the recombinant chimeric antigen may be a single-chain dimer structure.

In addition, preferably, the N-terminus of the recombinant chimeric antigen further comprises a signal peptide sequence; optionally, the signal peptide sequence is as shown in SEQ ID NO: 5;
Preferably, the C-terminus of the recombinant chimeric antigen further comprises a tag sequence. Optionally, the tag is at least one selected from the group consisting of a Flag tag, a His tag, an MBP tag, an HA tag, a myc tag, a GST tag, and a SUMO tag. Preferably, the tag is a His tag.

In the present application, to take into account the immune protection effects against both EEV and IMV virus particles, a recombinant chimeric antigen was designed. This antigen contains both the neutralizing antigen A35 of EEV and the neutralizing antigen M1 of IMV. The two neutralizing antigens are encoded by the A35R gene and M1R gene of the monkeypox virus, respectively. These genes are homologous to the A33R and L1R genes of vaccinia virus.

In a second aspect, the present application provides a preparation method of the recombinant chimeric antigen as described in the first aspect above, comprising the following steps:
adding a Kozak sequence and a coding sequence of a signal peptide to the 5' end and adding a coding sequence of a histidine tag and a stop codon to the 3' end of the nucleotide sequence encoding the recombinant chimeric antigen as described in the first aspect above, performing cloning and expression, screening for the correct recombinants, and then transfecting them into expression system cells for expression, collecting cell culture supernatant, and isolating the recombinant chimeric antigen peptide therefrom.

In a feasible implementation of the above preparation method, the cells of the expression system are mammalian cells, insect cells, yeast cells or bacterial cells;
optionally, the mammalian cell is a HEK293T cell, a 293F series cell or a CHO cell; further optionally, the 293F series cell is a HEK293F cell, a Freestyle293F cell or an Expi293F cell;
optionally, the insect cell is a sf9 cell, a Hi5 cell, a sf21 cell or a S2 cell; optionally, the yeast cell is a Pichia pastoris cell or a yeast cell transformed therefrom;
optionally, the bacterial cell is an Escherichia coli cell.

In a third aspect, the present application provides use of the recombinant chimeric antigen as described in the first aspect above for the preparation of a medicament for the prevention and/or treatment of poxvirus infection.

Optionally, the poxvirus is selected from the group consisting of Monkeypox virus, Cowpox virus, Variola virus and Vaccinia virus;
optionally, the medicament is a vaccine, preferably a recombinant protein vaccine; further preferably, the recombinant protein vaccine comprises an adjuvant selected from the group consisting of an aluminum adjuvant, MF59 adjuvant and an MF59-like adjuvant;
optionally, the vaccine is in the form of a nasal spray, an oral preparation, a suppository or a parenteral preparation;
preferably, the nasal spray is selected from the group consisting of an aerosol, a spray and a powder spray;
preferably, the oral preparation is selected from the group consisting of a tablet, a powder, a pill, a granule, a soft/hard capsule, a film-coated preparation and a paste; further preferably, the tablet is a sublingual tablet; further preferably, the granule is a fine granule; further preferably, the powder is a bulk powder; further preferably, the pill is a minipellet;
preferably, the parenteral preparation is selected from the group consisting of a transdermal preparation, an ointment, a plaster, a topical liquid, an injectable preparation; further preferably, the injectable preparation is a bolus-injectable preparation.

In a fourth aspect, the present application provides a vaccine or immunogenic composition, which comprises the recombinant chimeric antigen as described in the first aspect above, and a physiologically acceptable vehicle, adjuvant, excipient, carrier and/or diluent.

In some preferred specific embodiments, the vaccine or immunogenic composition is a poxvirus recombinant protein vaccine, which comprises the recombinant chimeric antigen and an adjuvant as described in the first aspect above.
Preferably, the adjuvant is one or more selected from the following adjuvants: an aluminum adjuvant, MF59 adjuvant and a MF59-like adjuvant.

In a feasible implementation, the vaccine or immunogenic composition is in the form of a nasal spray, an oral preparation, a suppository or a parenteral preparation;
preferably, the nasal spray is selected from the group consisting of aerosols, sprays and a powder spray;
preferably, the oral preparation is selected from the group consisting of a tablet, a powder, a pill, a granule, a soft/hard capsule, a film-coated preparations and a paste;
further preferably, the tablet is a sublingual tablet;
further preferably, the granule is a fine granule;
further preferably, the powder is a bulk powder;
further preferably, the pill is a minipellet;
preferably, the parenteral preparation is selected from the group consisting of transdermal preparations, ointments, plasters, topical liquids, injectable preparations; further preferably, the injectable preparation is a bolus-injectable preparation.

In a fifth aspect, the present application provides a method for preventing and/or treating poxvirus infection. The method comprises: administering to a subject in need thereof a preventive and/or therapeutically effective amount of the following substances: the recombinant chimeric antigen as described in the first aspect above, and/or the vaccine or immunogenic composition as described in the fourth aspect above.

The "preventively and/or therapeutically effective amount" may vary depending on the subject of administration, the subject's organ, symptoms, the method of administration, etc., and can be determined based on the doctor's judgment, taking into account the type of dosage form, the method of administration, the patient's age and weight, the patient's symptoms, etc.

### Beneficial Effects

The inventors of the present application have designed a recombinant chimeric antigen against poxvirus (especially the monkeypox virus), which comprises two immunogens arranged in a specific manner: the Monkeypox virus A35 protein or its antigenic fragment (or their derivative peptides), and the Monkeypox virus M1 protein or its antigenic fragment (or their derivative peptides). The former is a neutralizing antigen specific to intracellular mature virus particles (IMV), and the latter is a neutralizing antigen specific to extracellular enveloped virus particles (EEV). A vaccine comprising the two can stimulate an immune response against both types of infectious virus particles.

In a specific embodiment, the present application forms a single-chain tandem fusion multivalent antigen by directly connecting two Monkeypox virus A35 proteins or their antigenic fragments with two Monkeypox virus M1 proteins or their antigenic fragments in series or by connecting them in series through an appropriate linker sequence. In this tandem fusion multivalent antigen, A35 forms a stable dimer, which reverses the issue of poor immunogenicity when A35 protein is immunized alone. Meanwhile, the bivalency of M1 antigen significantly enhances its ability to elicit specific antibodies. Compared with immunizing the two proteins individually, the tandem fusion multivalent antigen not only retains the antigenicity of both antigens but also more efficiently activates specific protective antibodies against the monkeypox virus.

Compared with poxvirus vaccines in the prior art, the vaccine products based on the recombinant chimeric antigen of the present application have the following advantages:
1) Subunit vaccines have better safety, thus overcoming the safety issues associated with existing attenuated live virus vaccines. Additionally, compared with attenuated live virus vaccines, subunit vaccines have the advantages of low production cost, rapid response and production capacity support. Experimental verification shows that the poxvirus vaccine of the present application is highly effective;
2) Using the antigen sequence of the monkeypox virus itself, it has a high specificity for the monkeypox virus. Existing live virus vaccines are all developed based on vaccinia virus. Although the vaccinia virus and monkeypox virus belong to the same Poxviridae family, there are still certain differences in their neutralizing antigen sequences and antigenic epitopes, therefore, their protective effects against the monkeypox virus remain to be clarified. The poxvirus vaccine of the present application is developed based on the antigenic epitopes of the monkeypox virus, thus providing high specificity for the prevention and treatment of monkeypox virus;
3) The monkeypox virus contains many types of proteins, most of which cannot elicit an effective antiviral immune response, that is, they are ineffective components. Moreover, some viral proteins have immunosuppressive effects. Existing live virus vaccines cannot remove the above-mentioned ineffective and harmful components, which introduces risks and uncertainties in vaccination. In contrast, the poxvirus vaccine of the present application retains only two virus neutralizing antigens. Experimental data demonstrate that these two antigens alone can provide complete protective effects in mouse models.

### Description of the Drawings

One or more examples are exemplarily described by the figures in the corresponding drawings, and these exemplary descriptions do not constitute limitations on the examples. The word "exemplary" is used exclusively herein to mean "serving as an example, an embodiment, or an illustration". Any example described herein as "exemplary" is not necessarily to be construed as preferred or advantageous over other examples.
Figure 1 shows the gel filtration chromatography elution curves and SDS-PAGE identification results of the immunogenic proteins AA and DAM expressed in Example 2 of the present application.
Figure 2 shows the gel filtration chromatography elution curves and SDS-PAGE identification results of the single immunogenic proteins A35 and M1 expressed in Example 2 of the present application.
Figure 3 shows the results of affinity analysis of the immunogenic proteins M1 (A) and DAM (B) binding to the neutralizing antibody 7D11 detected in Example 3 of the present application.
Figure 4 shows the results of affinity analysis of the immunogenic proteins A35 (A), AA (B) and DAM (C) binding to the neutralizing antibody A27D7 detected in Example 3 of the present application.
Figure 5 is a schematic diagram of the mouse immunization and virus challenge strategy used in Examples 4 and 7 of the present application.
Figure 6 shows the specific binding antibody titers in the sera of immunized mice detected in Example 5 of the present application, wherein Figure 6A is the specific antibody level against each immunogen, and Figure 6B is the specific antibody level against the M1 and A35 epitopes.
Figure 7 shows the neutralizing antibody titer of the sera of immunized mice against live VACV virus detected in Example 6 of the present application.
Figure 8 shows the preliminary experimental results of BALB/c mice infected by intranasal drops of different doses of VACV-WR virus as described in Example 7 of the present application, wherein the horizontal axis shows the days after virus challenge and the vertical axis shows the probability of survival of mice.
Figure 9 shows the protective effect of each immunogenic protein recorded in Example 7 of the present application on BALB/c mice challenged with VACV-WR virus intranasally, wherein the horizontal axis shows the days after virus challenge, and the vertical axis shows the percent of weight change (A) and probability of survival (B) of the mice.
Figure 10 shows the results of the neutralization capacity assay of DAM - immunized mouse sera against live monkeypox virus (MPXV) described in Example 8 of the present application, with the VACV-VTT attenuated live vaccine immunized mouse serum as a control; wherein the horizontal axis shows the experimental group, and the vertical axis shows the MPXV-specific neutralizing antibody titer (PRNT₅₀).

### Detailed Description of the Invention

In order to make the aim, technical solutions, and advantages of the examples of the present application clearer, the technical solutions in the examples of the present application will be clearly and completely described below. Obviously, the described examples are only part of the examples of the present application, not all of the examples. Based on the examples in this application, all other examples obtained by ordinary technicians in this field without making any creative work shall fall within the scope of protection of this application.

In addition, in order to better illustrate the present application, numerous specific details are given in the following specific implementation method. It will be appreciated by those skilled in the art that the present application may be practiced without certain of the specific details. In some embodiments, raw materials, elements, methods, means, etc. well known to those skilled in the art are not described in detail in order to highlight the main purpose of the present application.

Unless explicitly stated otherwise, throughout the specification and claims, the term "comprise" or variations such as "include" or "comprising", etc., will be understood to include the stated elements or components but not to exclude other elements or components.

### Example 1: Immunogen design

In this example, as a representative example of the present application, two EEV neutralizing antigens A35 and two IMV neutralizing antigens M1 are connected in series in the arrangement of A35-M1-A35-M1 from N-terminus to C-terminus. The single-chain fusion peptide formed can assemble into a single-chain four-subunit structure of A35-A35 dimerization, hereinafter referred to as DAM, which represents the recombinant chimeric antigen of the present application.

The following sequences all use the sequence of the monkeypox virus isolate MPXV_USA_2022_MA001, which has the GenBank accession number ON563414.3 for its complete genome.

In the DAM, the two A35 neutralizing antigens use different A35 protein fragments. One is the S90-T181 peptide segment of the A35 protein (with the amino acid sequence as shown in SEQ ID NO:1), and the other is the S64-T181 peptide segment of the A35R protein (with the amino acid sequence as shown in SEQ ID NO:2). The amino acid sequences of the two M1 neutralizing antigens are identical, both having the amino acid sequence as shown in SEQ ID NO:3. Therefore, the DAM has the amino acid sequence as shown in SEQ ID NO:4.

During the construction of the DAM, to meet the needs of protein expression and purification, the inventors added a signal peptide sequence (as shown in SEQ ID NO:5) to the N-terminus of the DAM amino acid sequence as shown in SEQ ID NO:4 and added six histidine tags to the C-terminus, thereby forming a complete construct as shown in SEQ ID NO:6 below:
DAM complete construct (SEQ ID NO: 6)

METDTLLLWVLLLWVPGSTGSTTQYDHKESCNGLYYQGSCYILHSDYKSFEDAKANC
AAESSTLPNKSDVLTTWLIDYVEDTWGSDGNPITKTTSDYQDSDVSQEVRKYFCT
AAASIQTTVNTLSERISSKLEQEANASAQTKCDIEIGNFYIRQNHGCNITVKNMCSADADAQ
LDAVLSAATETYSGLTPEQKAYVPAMFTAALNIQTSVNTVVRDFENYVKQTCNSSAVVDNKL
KIQNVIIDECYGAPGSPTNLEFINTGSSKGNCAIKALMQLTTKATTQIAPRQVAGSANKAAI
TDSAVAVAAASSTHRKVVSSTTQYDHKESCNGLYYQGSCYILHSDYKSFEDAKAN
CAAESSTLPNKSDVLTTWLIDYVEDTWGSDGNPITKTTSDYQDSDVSQEVRKYFC
TAAASIQTTVNTLSERISSKLEQEANASAQTKCDIEIGNFYIRQNHGCNITVKNMCSADADA
QLDAVLSAATETYSGLTPEQKAYVPAMFTAALNIQTSVNTVVRDFENYVKQTCNSSAVVDNK
LKIQNVIIDECYGAPGSPTNLEFINTGSSKGNCAIKALMQLTTKATTQIAPRQVAGHHHHHH;

Among them, the first underlined part is the signal peptide sequence, the bold parts are the amino acid sequences of the two A35 peptide segments, the italic parts are the amino acid sequences of the two M1 peptide segments, and the last underlined part is the histidine tag sequence.

In addition, for comparison, in this example, an A35-A35 single-chain dimer structure (hereinafter referred to as AA) was also designed, which was formed by linking only two EEV neutralizing antigens A35 in series. In the construction of AA, the sequences were identical to those of the aforementioned DAM construct, except for the absence of the two M1 peptide segments. Similarly, to facilitate protein expression and purification, the inventors added a signal peptide sequence (as shown in SEQ ID NO:5) to its N-terminus and added six histidine tags to its C-terminus, thereby forming a complete construct as shown in SEQ ID NO:7 below:
AA complete construct (SEQ ID NO:7)

METDTLLLWVLLLWVPGSTGSTTQYDHKESCNGLYYQGSCYILHSDYKSFEDAKANC
AAESSTLPNKSDVLTTWLIDYVEDTWGSDGNPITKTTSDYQDSDVSQEVRKYFCT
SANKAAITDSAVAVAAASSTHRKVVSSTTQYDHKESCNGLYYQGSCYILHSDYKSFEDAKA
NCAAESSTLPNKSDVLTTWLIDYVEDTWGSDGNPITKTTSDYQDSDVSQEVRKYFCTHHHH
HH;

Among them, the first underlined part is the signal peptide sequence, the bold part is the amino acid sequence of the first A35 peptide segment, the italic part is the amino acid sequence of the second A35 peptide segment, and the last underlined part is the histidine tag sequence.

In addition, for comparison, in this example, expression constructs for the single antigen M1 and A35 peptide segments were also designed. The amino acid sequences of the M1 and A35 peptide segments are as follows:
Amino acid sequence of M1 peptide (SEQ ID NO: 8): 
MGAAASIQTTVNTLSERISSKLEQEANASAQTKCDIEIGNFYIRQNHGCNITVKNMC
SADADAQLDAVLSAATETYSGLTPEQKAYVPAMFTAALNIQTSVNTVVRDFENYVKQTCNSS
AVVDNKLKIQNVIIDECYGAPGSPTNLEFINTGSSKGNCAIKALMQLTTKATTQIAPRQVAG;
Amino acid sequence of A35 peptide (SEQ ID NO:9):

MSTTQYDHKESCNGLYYQGSCYILHSDYKSFEDAKANCAAESSTLPNKSDVLTTWL
IDYVEDTWGSDGNPITKTTSDYQDSDVSQEVRKYFCT.

### Example 2: Expression and purification of immunogenic protein

### Expression and purification of constructs DAM and AA

The amino acid sequences of the constructs DAM and AA designed in Example 1 were optimized for human codons to obtain nucleotide sequences encoding DAM and AA antigens, as shown in SEQ ID NO:10 and SEQ ID NO:11, respectively; these nucleotide sequences were added with a Kozak sequence (GCCACC) at the 5' end and a translation stop codon at the 3' end, and then these DNA fragments were artificially synthesized and cloned between the EcoRI and XhoI restriction sites of the pCAGGS vector, resulting in expression plasmids for the constructs DAM and AA.

The expression plasmids of DAM and AA were transfected into 293F cells respectively for in vitro recombinant expression; 5-7 days after transfection, the cell supernatant containing the expressed immunogenic protein was collected. The protein was then purified as follows: The cell supernatant containing the immunogenic protein was subjected to crude purification using nickel ion affinity chromatography (HisTrapTM HP (GE)), and then further purified using a gel filtration chromatography column (Superdex 200 10/300GL (GE)), and finally, the protein purity and molecular weight were identified by SDS-PAGE.

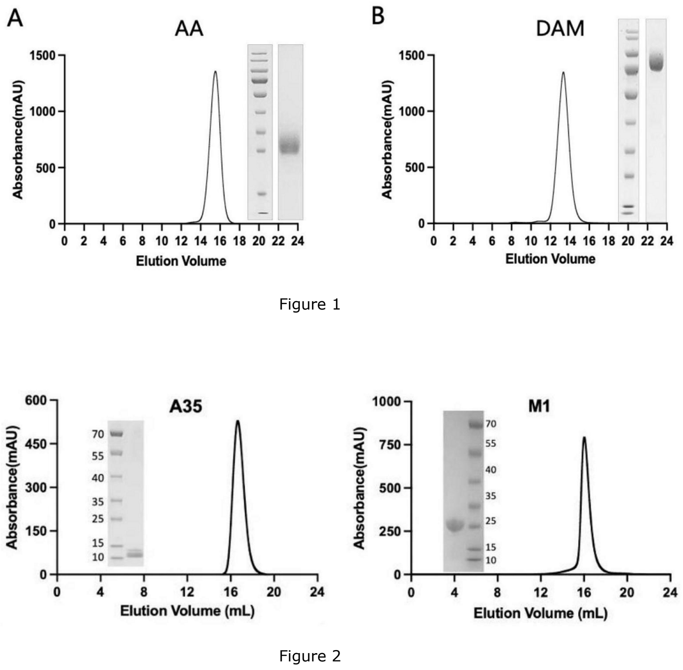

The elution curve of gel filtration chromatography and the SDS-PAGE identification results are shown in Figure 1. Figure 1 shows that after two-step purification, high-purity AA and DAM proteins were obtained with molecular weights of ~25 kDa and ~75 kDa, respectively, which were in line with expectations.

### Expression and purification of single immunogens M1 and A35

The amino acid sequences of the single antigen M1 and A35 peptides designed in Example 1 were subjected to human codon optimization to obtain nucleotide sequences encoding the M1 and A35 peptides, as shown in SEQ ID NO: 12 and SEQ ID NO: 13, respectively. A translation stop codon was added to the 3' end of these nucleotide sequences, and then these DNA fragments were artificially synthesized and cloned into the pET-28a expression vector to obtain expression plasmids of M1 and A35.

The expression plasmids of M1 and A35 were expressed using the Escherichia coli (E. coli) system, and the resulting inclusion bodies were renatured in vitro into active proteins M1 and A35 using arginine dilution renaturation method. Then, the renatured M1 or A35 protein was purified using a gel filtration chromatography column Superdex200 100/300GL, and finally, the protein purity and molecular weight were identified by SDS-PAGE.

The elution curve of gel filtration chromatography and the SDS-PAGE identification results are shown in Figure 2. As can be seen from Figure 2, high-purity M1 and A35 proteins can be obtained after the above-mentioned gel filtration chromatography purification. The peak positions of the M1 protein and the SDS-PAGE both indicated a molecular weight of ~20kDa, which were consistent with expectations. The peak position of the A35 protein corresponded to a molecular weight of 25kDa, while SDS-PAGE showed a molecular weight of ~12kDa, indicating that the A35 protein is a dimer formed by intermolecular interactions, which is consistent with previous literature reports.

### Example 3: Antigenicity detection of immunogens

In order to detect the exposure and antigenicity of each antigenic epitope of the recombinant chimeric antigen of the present application, in this example, the ability of the immunogenic proteins expressed and purified in Example 2 (including single-chain fusion immunogenic proteins DAM and AA, as well as single antigenic proteins M1 and A35) to bind to neutralizing antibodies 7D11 and A27D7 was detected using the surface plasmon resonance (SPR) experimental method. The 7D11 and A27D7 antibodies are neutralizing antibodies for vaccinia virus antigens L1 and A33, respectively. Data indicate that they can cross-recognize the M1 and A35 antigens of monkeypox virus, among which the A27D7 antibody can recognize the dimer epitope of A35.

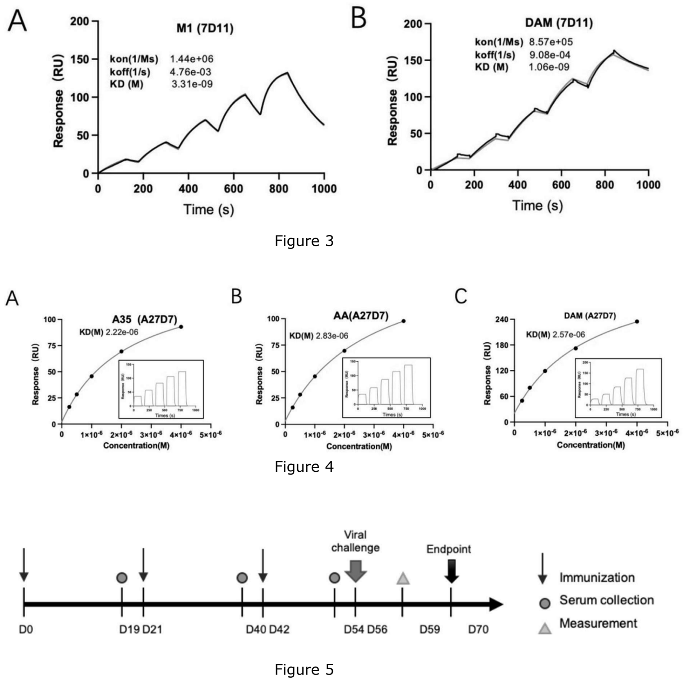

The results are shown in Figures 3 and 4.

As can be seen from Figures 3A and 3B, the single antigen M1 had an affinity of 3.3 nM for the neutralizing antibody 7D11, exhibiting a slow binding and slow dissociation pattern, while the recombinant chimeric antigen DAM in the present application had an affinity for 7D11 that is three times higher than that of M1. This was manifested by an increase in the binding rate (kon) and a decrease in the dissociation rate (koff), indicating that the bivalent M1 epitopes on DAM were exposed and can be recognized by specific antibodies. Moreover, the bivalency of M1 increased the number of M1 epitopes on DAM, thereby enhancing its binding ability and antigenicity for the antibody.

As can be seen from Figures 4A-C, the single antigen A35 had an affinity of 2.2 µM for the antibody A27D7, exhibiting a fast binding and fast dissociation pattern. The single-chain fusion immunogen AA had an affinity for antibody A27D7 that was similar to that of the single antigen A35. The recombinant chimeric antigen DAM in the present application also had an affinity for antibody A27D7 that was similar to the above two, indicating that the A35 epitopes on DAM were exposed and present in a stable dimeric form.

The above data all indicate that the A35 and M1 antigen epitopes on the recombinant chimeric antigen DAM of the present application are well exposed and have high antigenicity. In particular, the bivalency of the M1 epitope greatly enhances its antigenicity compared with the single antigen protein M1.

### Example 4: Mouse immunization experiment

In order to verify the immune protection efficacy of the recombinant chimeric antigen of the present application, the inventors mixed and emulsified each immunogenic protein obtained in Example 2 with AddavVax^{™} adjuvant, and immunized mice according to the strategy shown in Figure 5. The replication-competent vaccinia virus Tiantan strain (VACV-VTT) was used as the attenuated live vaccine control group.

In the immunization experiment, all mice used were female BALB/c mice, 6-8 weeks old, with an average body weight of 15-20 g. Six mice were used in each experimental group. As shown in Figure 4, mice were immunized on Day 0, Day 21 and Day 42, for a total of 3 times, with each dose of 10 µg/mouse. The vaccination method was intramuscular injection, with the injection site located in the thigh of the mouse, and 50 µL was injected into each leg.

The VACV-VTT group was immunized by scarification at the base of the tail. Specifically, the mice were immunized once on Day 0 with a dose of 107 PFU/mouse.

Two days before the second immunization, the third immunization, and the virus challenge (that is, on Day 19, Day 40, and Day 54), orbital blood was collected from mice in all groups. Mouse serum was obtained by centrifugation at 1500 rpm for 10 minutes after static coagulation. The mouse serum was immediately packaged and stored in a -80°C refrigerator for subsequent ELISA detection of specific antibody titers and determination of live virus neutralization ability.

### Example 5: ELISA test to detect the specific antibody titer induced by the vaccine

Each immunogenic protein used for mouse immunization (i.e., single antigenic proteins A35 and M1, single-chain fusion immunogens AA and DAM, prepared in Example 2) was diluted to 2 µg/mL with ELISA coating solution (Solarbio, C1050). 100 µL of the diluted immunogenic protein or the vaccinia Tiantan strain-infected cell lysate (for antibody titer detection of the attenuated live vaccine immunization group) was added to each well of a 96-well ELISA plate (Corning, 3590), and allowed to stand overnight at 4°C. The coating solution was discarded, and the wells were washed with PBS to remove residual coating solution. 100 µL of ELISA blocking solution (10% skim milk powder prepared in PBST) was added, and allowed to stand at room temperature for 1 hour for blocking. During the blocking period, the immune mouse serum was diluted with the ELISA blocking buffer, starting from a 200-fold dilution and then performing 3-fold serial dilutions, with a total of 11 dilution steps for each sample. After the blocking was completed, the blocking buffer was removed, and the immune mouse serum diluted 10-fold with the blocking buffer was added to the ELISA plate, with 100 µL for each dilution degree. The plate was then incubated at room temperature for 1 hour, followed by washing with PBST for 3 times. Then, HRP-labeled goat anti-mouse secondary antibody (Abcam, ab6789), diluted 1:4000 with blocking solution, was added and incubated at room temperature for 1 hour, and then washed 5-6 times with PBST. TMB substrate solution was added for color development, and the reaction was terminated with 2M HCl after an appropriate period of time. The OD₄₅₀ absorbance was measured using a microplate reader. A sample was considered positive if its OD₄₅₀ value was more than 2.5 times that of the negative control. The highest dilution of the serum at which the sample was still positive was defined as the serum antibody titer (Endpoint titer). If the reaction value of the lowest dilution was still less than 2.5 times that of the negative control, the titer of the sample was defined as half of the lowest dilution, that is, Log10=1.

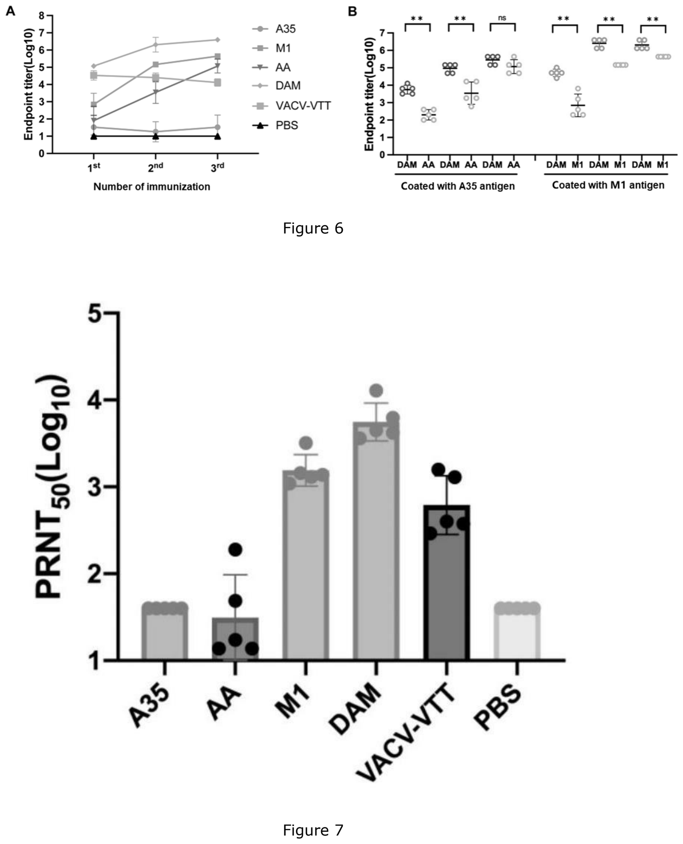

The detection results of the serum specific antibody titer of mice immunized with each immunogen protein are shown in Figure 6, wherein Figure 6A shows the specific antibody levels for each immunogen, and Figure 6B shows the specific antibody levels for the M1 and A35 epitopes. As shown in Figure 6A, the single-chain fusion antigen DAM of the present application could effectively elicit specific antibodies after the first immunization. Moreover, the specific antibody titers after each immunization were better than those of all control groups, including the attenuated live vaccine immunization. It is worth noting that, with respect to the A35 epitope, the A35 protein alone could not elicit specific antibodies and lacked immunogenicity, while the single-chain fusion modified AA and DAM could effectively elicit specific antibody responses against the A35 epitope and exhibited good immunogenicity (Figure 6B). Furthermore, DAM could simultaneously elicit high levels of specific antibody responses against both the A35 and M1 epitopes, with levels higher than that of the single immunization groups (AA and M1, Figure 6B).

### Example 6: Determination of the neutralizing ability of immune serum against live VACV virus

Vaccinia virus (VACV) is a model virus of the poxvirus genus. Due to the high homology between the main immunogens of the poxvirus genus, vaccinia virus which has a lower biosafety level and is easier to manipulate, is commonly used internationally to evaluate the neutralization ability of other poxvirus vaccines at the cellular level and to assess the protective efficacy in mouse models. Therefore, in this example, the mouse-adapted vaccinia virus strain Western Reserve (VACV-WR), which is commonly used internationally, was used to determine the live virus neutralizing ability of each immunogenic protein at the cellular level.

The immune mouse serum obtained in Example 4 was diluted with a culture medium (DMEM) containing 2% inactivated serum, starting from a 20-fold dilution, followed by 2-fold serial dilutions, resulting in a total of 10 dilution steps for each sample. The VACV-WR virus was also diluted to 500 PFU/mL using the same diluent. 200 µL of the diluted immune mouse serum (with the diluent without immune mouse serum used as the control well) was mixed with 200 µL of the diluted virus solution and incubated at 37°C for 1 hour. Vero cells were seeded in a 12-well plate one day in advance, and a confluency of approximately 95% on the second day was considered optimal. The culture medium in the 12-well plate was discarded, and the residual culture medium was washed away with PBS. The incubated serum-virus mixture was added to the 12-well plate and infected at 37°C for 2 hours. After infection, the virus-serum mixture was removed, and the residual virus was washed off with PBS. A carboxymethyl cellulose-DMEM mixture prepared (by mixing 2% carboxymethyl cellulose and 2×DMEM at a ratio of 1:1) was added and cultured at 37°C for 48-60 hours. After observing distinct CPE under the microscope, the cells were fixed by adding 4% paraformaldehyde fix solution at room temperature for 2 hours, followed by crystal violet staining and counting.

The calculation method of PRNT₅₀ was as follows: Wells containing only the virus were used as control wells. The inhibition rate of the virus by the serum in each experimental well was calculated by dividing the number of plaques in the experimental well by the number of plaques in the control wells. Subsequently, the PRNT₅₀ was calculated using the formula log(inhibitor) vs. normalized response -- Variable slope in the GraphPad software.

The results are shown in Figure 7.

Since the VACV-WR strain used in the live virus neutralization assay rarely produces EEV, we used IMV in our neutralization experiments.
As shown in Figure 7, A35 is absent in IMV virus particles. Therefore, the A35 and AA immunization groups did not exhibit neutralizing ability against the live viruses. In contrast, the M1, DAM and VACV-VTT immunization groups all produced neutralizing antibodies against IMV virus particles, among which the DAM group elicited a significantly higher level of neutralizing antibodies compared to the M1 and the attenuated live vaccine VACV-VTT immunization groups.

### Example 7: VACV challenge protection experiment

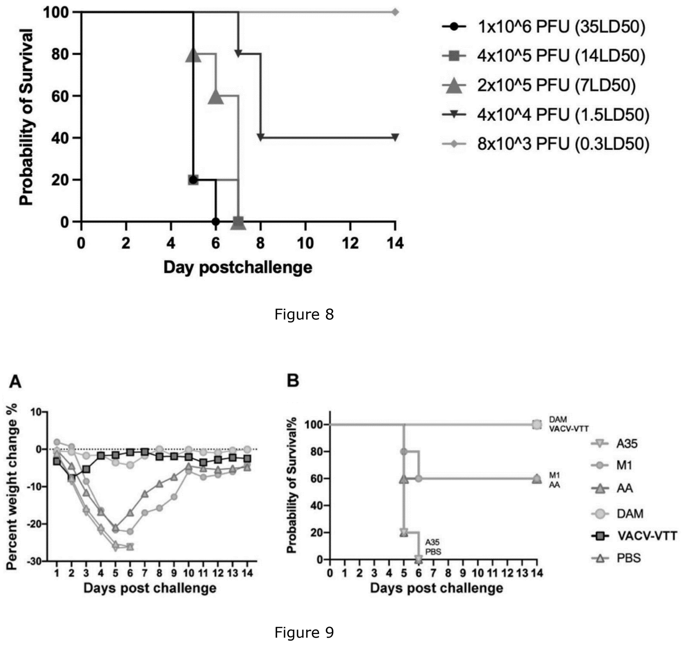

Intranasal infection of BALB/c mice with the VACV-WR strain can lead to death. To determine the appropriate challenge dose, we first conducted a preliminary experiment of intranasal infection with different doses of VACV-WR virus in 17- to 19-week-old BALB/c mice (consistent with the age of mice at challenge after three immunizations), to determine the LD50 of VACV-WR of the infected BALB/c mice in our experimental system. The preliminary experimental results are shown in Figure 8. When the challenge dose was 2×10⁵ PFU, all mice died within 7 days after infection. By calculation, this virus dose was 7LD₅₀. We subsequently used this virus dose as the challenge dose for our animal experiments.

As shown in Figure 5, in Example 4, mice that had received three immunizations were subjected to intranasal challenge with 7LD50 of VACV-WR virus two weeks after the third immunization (on day 56), to evaluate the protective efficacy of the vaccine in the animal model. The results of the percentage change in body weight and the survival percentage of mice in each challenged experimental group are shown in Figures 9A and 9B, respectively.

The results of Figures 9A and 9B indicate that the single antigen A35 immunization group had no protective effect on mice infected with the VACV-WR virus. All mice died within 6 days after challenge, consistent with its inability to effectively elicit specific antibodies. Although the single antigen M1 and the single-chain fusion immunogen AA exhibited high levels of specific antibodies in Example 4 and exhibited high levels of neutralizing antibody elicitation ability in Example 5, they only protected 60% of the mice from lethal VACV-WR infection, and the surviving mice still showed significant weight loss. Compared with the above groups, the single-chain fusion immunogen DAM of the present application can provide 100% protection against VACV-WR infection in mice, with no significant change in body weight.

The above results indicate that the single-chain fusion immunogen DAM of the present application exhibits an excellent protective effect in the mouse model.

### Example 8: Determination of the neutralizing ability of immune serum against live monkeypox virus (MPXV)

To further determine the neutralizing ability of the DAM immunogen against MPXV, mice serum immunized three times with the DAM immunogen and mice serum immunized with the VACV-VTT attenuated live vaccine (as a control) obtained in Example 4 were diluted with a culture medium (DMEM) containing 2% inactivated serum, starting from a 20-fold dilution, followed by 2-fold serial dilutions, resulting in a total of 10 dilution steps for each sample. The MPXV virus was also diluted to 500 PFU/mL using the same diluent. 200 µL of the diluted immune mouse serum (with an equal volume of PBS used to replace the immune mouse serum dilution solution to serve as the control well) was mixed with 200 µL of the diluted virus solution and incubated at 37°C for 1 hour. Vero cells were seeded in a 12-well plate one day in advance, and a confluency of approximately 95% on the second day was considered optimal. The culture medium in the 12-well plate was discarded, and the residual culture medium was washed away with PBS. The incubated serum-virus mixture was added to the 12-well plate and infected at 37°C for 2 hours. After infection, the virus-serum mixture was removed, and the residual virus was washed off with PBS. A carboxymethyl cellulose-DMEM mixture prepared (by mixing 2% carboxymethyl cellulose and 2×DMEM at a ratio of 1:1) was added and cultured at 37°C for 48-60 hours. After observing distinct CPE under the microscope, the cells were fixed by adding 4% paraformaldehyde fix solution at room temperature for 2 hours, followed by crystal violet staining and counting.

The calculation method of PRNT₅₀ was as follows: Wells containing only the virus were used as control wells. The inhibition rate of the virus by the serum in each experimental well was calculated by dividing the number of plaques in the experimental well by the number of plaques in the control wells. Subsequently, the PRNT50 was calculated using the formula log(inhibitor) vs. normalized response -- Variable slope in the GraphPad software.

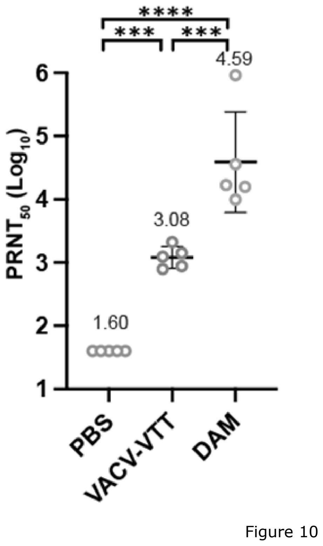

The results are shown in Figure 10. As shown in Figure 10, the ability of DAM immunogen to elicit the production of MPXV-specific neutralizing antibodies was significantly higher than that of the attenuated live vaccine VACV-VTT immunization group, with a PRNT₅₀ of 38797 (10^{4.59}), which was 1.5 times that of the VACV-VTT immunization group.

In summary, the DAM immunogen of the present application has excellent immunogenicity and immune protection effect, and has great potential in the development and application of poxvirus vaccines represented by monkeypox virus.

Finally, it should be noted that the above examples are only used to illustrate the technical solutions of the present application, rather than to limit it. Although the present application has been described in detail with reference to the aforementioned examples, those skilled in the art should understand that they can still modify the technical solutions described in the aforementioned examples, or make equivalent replacements for some of the technical features therein. However, these modifications or replacements do not deviate the essence of the corresponding technical solutions from the spirit and scope of the technical solutions of the present application.

### Industrial Applicability

The recombinant chimeric antigen for poxvirus of the present application can efficiently elicit specific immune protection effects against monkeypox virus, and has good safety, rapid responsiveness and production capacity support. Therefore, it has excellent clinical application prospects and industrialization value.

### Sequences involved in the present application:

SEQ ID NO:1(the amino acid sequence of the first A35 peptide segment in DAM) 
STTQYDHKESCNGLYYQGSCYILHSDYKSFEDAKANCAAESSTLPNKSDVLTTWLI
DYVEDTWGSDGNPITKTTSDYQDSDVSQEVRKYFCT
SEQ ID NO:2(the amino acid sequence of the second A35 peptide segment in DAM) 
SANKAAITDSAVAVAAASSTHRKVVSSTTQYDHKESCNGLYYQGSCYILHSDYKS
FEDAKANCAAESSTLPNKSDVLTTWLIDYVEDTWGSDGNPITKTTSDYQDSDVSQ
EVRKYFCT
SEQ ID NO:3(the amino acid sequence of the M1 peptide segment in DAM) 
AAASIQTTVNTLSERISSKLEQEANASAQTKCDIEIGNFYIRQNHGCNITVKNMCSADADAQ
LDAVLSAATETYSGLTPEQKAYVPAMFTAALNIQTSVNTVVRDFENYVKQTCNSSAVVDNKL
KIQNVIIDECYGAPGSPTNLEFINTGSSKGNCAIKALMQLTTKATTQIAPRQVAG
SEQ ID NO:4 (the amino acid sequence of DAM) 
STTQYDHKESCNGLYYQGSCYILHSDYKSFEDAKANCAAESSTLPNKSDVLTTWLI
DYVEDTWGSDGNPITKTTSDYQDSDVSQEVRKYFCTAAASIQTTVNTLSERISSKLEQ
EANASAQTKCDIEIGNFYIRQNHGCNITVKNMCSADADAQLDAVLSAATETYSGLTPEQKAY
VPAMFTAALNIQTSVNTVVRDFENYVKQTCNSSAVVDNKLKIQNVIIDECYGAPGSPTNLEFI
NTGSSKGNCAIKALMQLTTKATTQIAPRQVAGSANKAAITDSAVAVAAASSTHRKVVS
STTQYDHKESCNGLYYQGSCYILHSDYKSFEDAKANCAAESSTLPNKSDVLTTWLI
DYVEDTWGSDGNPITKTTSDYQDSDVSQEVRKYFCTAAASIQTTVNTLSERISSKLEQ
EANASAQTKCDIEIGNFYIRQNHGCNITVKNMCSADADAQLDAVLSAATETYSGLTPEQKAY
VPAMFTAALNIQTSVNTVVRDFENYVKQTCNSSAVVDNKLKIQNVIIDECYGAPGSPTNLEFI
NTGSSKGNCAIKALMQLTTKATTQIAPRQVAG
SEQ ID NO:5 (the amino acid sequence of the signal peptide)
   METDTLLLWVLLLWVPGSTG
SEQ ID NO:6 (the amino acid sequence of the complete DAM construct in Example 1) 
METDTLLLWVLLLWVPGSTGSTTQYDHKESCNGLYYQGSCYILHSDYKSFEDAKANC
AAESSTLPNKSDVLTTWLIDYVEDTWGSDGNPITKTTSDYQDSDVSQEVRKYFCT
AAASIQTTVNTLSERISSKLEQEANASAQTKCDIEIGNFYIRQNHGCNITVKNMCSADADAQ
LDAVLSAATETYSGLTPEQKAYVPAMFTAALNIQTSVNTVVRDFENYVKQTCNSSAVVDNKL
KIQNVIIDECYGAPGSPTNLEFINTGSSKGNCAIKALMQLTTKATTQIAPRQVAGSANKAAI
TDSAVAVAAASSTHRKVVSSTTQYDHKESCNGLYYQGSCYILHSDYKSFEDAKAN
CAAESSTLPNKSDVLTTWLIDYVEDTWGSDGNPITKTTSDYQDSDVSQEVRKYFC
TAAASIQTTVNTLSERISSKLEQEANASAQTKCDIEIGNFYIRQNHGCNITVKNMCSADADA
QLDAVLSAATETYSGLTPEQKAYVPAMFTAALNIQTSVNTVVRDFENYVKQTCNSSAVVDNK
LKIQNVIIDECYGAPGSPTNLEFINTGSSKGNCAIKALMQLTTKATTQIAPRQVAGHHHHHH
SEQ ID NO:7 (the amino acid sequence of the complete AA construct in Example 1) 
METDTLLLWVLLLWVPGSTGSTTQYDHKESCNGLYYQGSCYILHSDYKSFEDAKANC
AAESSTLPNKSDVLTTWLIDYVEDTWGSDGNPITKTTSDYQDSDVSQEVRKYFCT
SANKAAITDSAVAVAAASSTHRKVVSSTTQYDHKESCNGLYYQGSCYILHSDYKSFEDAKA
NCAAESSTLPNKSDVLTTWLIDYVEDTWGSDGNPITKTTSDYQDSDVSQEVRKYFCTHHHH
HH
SEQ ID NO:8 (the amino acid sequence of the single antigen M1 peptide segment in Example 1) 
MGAAASIQTTVNTLSERISSKLEQEANASAQTKCDIEIGNFYIRQNHGCNITVKNMCSADAD
AQLDAVLSAATETYSGLTPEQKAYVPAMFTAALNIQTSVNTVVRDFENYVKQTCNSSAVVDN
KLKIQNVIIDECYGAPGSPTNLEFINTGSSKGNCAIKALMQLTTKATTQIAPRQVAG
SEQ ID NO:9 (the amino acid sequence of the single antigen A35 peptide segment in Example 1) 
MSTTQYDHKESCNGLYYQGSCYILHSDYKSFEDAKANCAAESSTLPNKSDVLTTWLIDYVE
DTWGSDGNPITKTTSDYQDSDVSQEVRKYFCT
SEQ ID NO:10 (the nucleotide sequence encoding the complete DAM construct as shown in SEQ ID NO: 6) 
ATGGAAACAGACACCCTGCTGCTGTGGGTCCTGCTGCTCTGGGTGCCTGGCAGCACCGG
CAGCACCACCCAGTACGACCACAAAGAGAGCTGCAACGGCCTGTACTACCAAGGCTCTT
GTTACATCCTGCACAGCGACTATAAGAGCTTCGAGGATGCTAAGGCCAATTGCGCCGCC
GAGAGCAGCACACTGCCTAACAAGTCCGACGTGCTGACCACCTGGCTGATCGACTACGT
GGAAGATACATGGGGAAGCGACGGCAACCCCATCACAAAAACCACAAGCGATTATCAGG
ACAGCGACGTGTCTCAGGAGGTTAGAAAGTACTTTTGCACCGCCGCCGCCTCTATCCAG
ACCACAGTGAACACCCTGAGCGAGCGGATCAGCTCCAAGCTGGAGCAGGAGGCAAACG
CAAGCGCCCAGACAAAGTGCGACATCGAGATCGGCAATTTCTACATCAGACAGAACCAC
GGCTGCAATATCACCGTGAAGAATATGTGCTCCGCCGACGCAGATGCACAGCTGGATGC
CGTGCTGTCCGCCGCAACCGAGACATACTCTGGCCTGACCCCTGAGCAGAAGGCCTATG
TGCCAGCCATGTTCACAGCCGCCCTGAACATCCAGACCAGCGTGAATACAGTGGTGCGG
GACTTTGAGAACTACGTGAAGCAGACCTGCAATTCTAGCGCCGTGGTGGACAACAAGCT
GAAGATCCAGAATGTGATCATCGATGAGTGTTATGGAGCACCAGGATCCCCTACCAACCT
GGAGTTCATCAATACAGGCTCCTCTAAGGGCAACTGCGCCATCAAGGCCCTGATGCAGC
TGACCACAAAGGCCACCACACAGATCGCACCTAGGCAGGTGGCAGGATCCGCCAATAAG
GCAGCCATCACCGACTCTGCCGTGGCAGTGGCAGCAGCCAGCTCCACACACCGCAAGGT
GGTGTCTAGCACCACACAGTACGATCACAAGGAATCCTGTAATGGCCTGTACTATCAGGG
CTCCTGTTACATCCTGCACTCTGACTATAAGAGCTTTGAGGATGCCAAGGCCAACTGTGC
CGCCGAGTCCTCTACCCTGCCAAATAAGAGCGACGTGCTGACCACATGGCTGATCGACT
ATGTGGAGGATACCTGGGGCTCTGATGGCAACCCCATCACAAAGACCACATCTGATTACC
AGGACAGCGACGTGAGCCAGGAGGTGCGGAAGTATTTCTGCACCGCTGCTGCTTCCATC
CAGACCACAGTGAATACACTGAGCGAGAGAATCAGCTCCAAACTGGAGCAGGAGGCCAA
 
CGCCTCTGCCCAGACCAAGTGTGACATCGAGATCGGCAACTTCTACATCAGGCAGAATCA
TGGATGTAATATCACCGTGAAGAACATGTGCTCCGCCGATGCCGACGCCCAGCTGGACG
CCGTGCTGAGCGCCGCCACTGAGACATACTCCGGCCTGACCCCCGAACAGAAAGCCTAT
GTGCCTGCTATGTTTACCGCCGCCCTGAACATTCAGACCAGCGTCAACACAGTGGTGCG
CGATTTCGAAAATTATGTCAAACAGACTTGTAATTCTAGCGCCGTCGTCGATAACAAGCT
GAAGATTCAGAATGTGATTATTGACGAATGCTACGGAGCACCAGGATCCCCAACCAACCT
GGAGTTTATCAACACCGGCTCCTCTAAGGGCAATTGTGCCATCAAGGCCCTGATGCAACT
GACTACCAAAGCTACCACACAGATCGCACCAAGGCAGGTGGCAGGACACCACCACCACC
ACCAC
SEQ ID NO:11 (the nucleotide sequence encoding the complete AA construct as shown in SEQ ID NO: 7) 
ATGGAAACAGACACCCTGCTGCTGTGGGTCCTGCTGCTCTGGGTGCCTGGCAGCACCGG
CAGCACCACCCAGTACGACCACAAAGAGAGCTGCAACGGCCTGTACTACCAAGGCTCTT
GTTACATCCTGCACAGCGACTATAAGAGCTTCGAGGATGCTAAGGCCAATTGCGCCGCC
GAGAGCAGCACACTGCCTAACAAGTCCGACGTGCTGACCACCTGGCTGATCGACTACGT
GGAAGATACATGGGGAAGCGACGGCAACCCCATCACAAAAACCACAAGCGATTATCAGG
ACAGCGACGTGTCTCAGGAGGTTAGAAAGTACTTTTGCACCAGCGCCAACAAAGCCGCT
ATCACCGACAGCGCCGTGGCCGTGGCCGCCGCATCTAGCACCCACAGAAAGGTGGTGTC
CTCCACAACACAGTACGACCACAAGGAAAGCTGTAATGGACTGTACTACCAGGGCAGCT
GCTACATCCTGCACAGCGACTACAAGTCTTTCGAGGACGCCAAGGCCAACTGCGCCGCT
GAGTCTAGCACCCTGCCAAACAAGAGCGATGTGCTGACAACCTGGCTGATTGACTACGT
GGAAGATACCTGGGGCTCCGATGGCAACCCTATCACCAAGACCACATCTGACTACCAGG
ATAGCGACGTGTCCCAGGAGGTGCGGAAGTACTTCTGCACCCACCACCACCACCACCAT
SEQ ID NO:12 (the nucleotide sequence encoding the single antigen M1 peptide segment (i.e., SEQ ID NO:8)) 
atgggagcagctgcgtcaatacaaacaactgtaaacaccctgagcgaacgtattagctccaaacttgagcaagag
gcaaacgcgagcgcgcaaacgaaatgcgatattgagatcggcaacttctatatccgccaaaatcacggttgtaata
ttaccgtcaagaacatgtgcagcgcggacgcggacgcgcagctggacgccgttttgtctgcagcgaccgaaaccta
ttccggtctgaccccggagcagaaagcgtacgttccggctatgttcaccgcagcactcaatatccaaaccagcgtca
ataccgttgttcgtgattttgaaaattacgtgaagcagacgtgcaactcctcggcggtggtggataacaaactgaag
atccaaaacgtgattatcgacgaatgttacggcgctccgggttctccgaccaacttggagtttatcaacactggcagc
agcaaaggcaactgcgctattaaggcgctgatgcagctgactacaaaggcgaccacgcagatcgccccacgtcag
gtggccggt
SEQ ID NO:13 (the nucleotide sequence encoding the single antigen A35 peptide segment (i.e., SEQ ID NO:9))

atgtcaactacacaatatgatcacaaagaaagttgcaacggcttatattatcagggttcttgttatatcctgcacagcg
actacaagtcttttgaggatgcgaaagctaactgcgcggcagaaagcagcaccctgccgaataagtccgacgtgtt
gaccacgtggctgatcgattacgtggaagatacctggggttccgacggcaacccgattaccaaaactacgagcgac
taccaggatagcgacgtttcgcaagaggttcgtaaatacttctgcacc

## Claims

1. A recombinant chimeric antigen for poxvirus, **characterized in that** the recombinant chimeric antigen comprises an amino acid sequence arranged according to the following formula (I):
A1-C₁-M1-C₂-A2-C₃-M2 (I)
In formula (I):
A1 represents Monkeypox virus A35 protein or an antigenic fragment I thereof, or an amino acid sequence having at least 90%, 92%, 95%, 96%, 97%, 98% or 99% identity thereto and having the same or substantially the same immunogenicity thereto,
A2 represents Monkeypox virus A35 protein or an antigenic fragment II thereof, or an amino acid sequence having at least 90%, 92%, 95%, 96%, 97%, 98% or 99% identity thereto and having the same or substantially the same immunogenicity thereto,
M1 represents Monkeypox virus M1 protein or an antigenic fragment I thereof, or an amino acid sequence having at least 90%, 92%, 95%, 96%, 97%, 98% or 99% identity thereto and having the same or substantially the same immunogenicity thereto,
M2 represents Monkeypox virus M1 protein or an antigenic fragment II thereof, or an amino acid sequence having at least 90%, 92%, 95%, 96%, 97%, 98% or 99% identity thereto and having the same or substantially the same immunogenicity thereto;
C1 , C2 , C3 are each independently absent, or a linking sequence (GGGGS)n, where n is any integer between 1 and 10;
wherein,
A1 and A2 are the same or different,
M1 and M2 are the same or different.

2. The recombinant chimeric antigen for poxvirus according to claim 1, **characterized in that** the antigenic fragment I or II of the A35 protein is an extracellular segment of the protein or a part thereof;
and/or, the antigenic fragment I or II of the M1R protein is an extracellular segment of the protein or a part thereof.

3. The recombinant chimeric antigen for poxvirus according to claim 1 or 2, **characterized in that** A1 represents the amino acid sequence as shown in SEQ ID NO:1, or an amino acid sequence having the same or substantially the same immunogenicity thereto obtained by substituting, deleting or adding one or more amino acids to the amino acid sequence as shown in SEQ ID NO: 1;
and/or, A2 represents the amino acid sequence as shown in SEQ ID NO:1, or an amino acid sequence of the amino acid sequence as shown in SEQ ID NO:1 with a fragment of 1-30 amino acids extended to the N-terminus of the A35 protein, or an amino acid sequence having the same or substantially the same immunogenicity thereto obtained by substituting, deleting or adding one or more amino acids to the above amino acid sequences; preferably, A2 represents the amino acid sequence as shown in SEQ ID NO:2, or an amino acid sequence having the same or substantially the same immunogenicity thereto obtained by substituting, deleting or adding one or more amino acids to the amino acid sequence as shown in SEQ ID NO: 2;
and/or, M1 and M2 are the same and represent the amino acid sequence as shown in SEQ ID NO:3, or an amino acid sequence having the same or substantially the same immunogenicity thereto obtained by substituting, deleting or adding one or more amino acids to the amino acid sequence as shown in SEQ ID NO: 3.

4. The recombinant chimeric antigen for poxvirus according to claim 3, **characterized in that** A1 represents the amino acid sequence as shown in SEQ ID NO: 1, A2 represents the amino acid sequence as shown in SEQ ID NO: 1 or SEQ ID NO: 2, and M1 and M2 are the same and represent the amino acid sequence as shown in SEQ ID NO: 3;
preferably, the C1 , C2 , C3 are all absent;
further preferably, the recombinant chimeric antigen comprises the amino acid sequence as shown in SEQ ID NO:4;
optionally, the recombinant chimeric antigen is a single-chain dimer structure.

5. The recombinant chimeric antigen for poxvirus according to any one of claims 1-4, **characterized in that** the N-terminus of the recombinant chimeric antigen further comprises a signal peptide sequence; optionally, the signal peptide sequence is as shown in SEQ ID NO:5;
and/or, the C-terminus of the recombinant chimeric antigen further comprises a tag sequence; optionally, the tag is at least one selected from the group consisting of a Flag tag, a His tag, a MBP tag, a HA tag, a myc tag, a GST tag, and a SUMO tag, and preferably a His tag.

6. A preparation method of the recombinant chimeric antigen according to any one of claims 1-5, comprising the following steps: adding a Kozak sequence and a coding sequence of a signal peptide to the 5' end and adding a coding sequence of a histidine tag and a stop codon to the 3' end of the nucleotide sequence encoding the recombinant chimeric antigen according to any one of claims 1 to 5, performing cloning and expression, screening for the correct recombinants, and then transfecting them into expression system cells for expression, collecting cell culture supernatant, and isolating the recombinant chimeric antigen peptide therefrom.

7. The preparation method according to claim 6, **characterized in that** the expression system cell is a mammalian cell, an insect cell, a yeast cell or a bacterial cell;
optionally, the mammalian cell is a HEK293T cell, a 293F series cell or a CHO cell; further optionally, the 293F series cell is a HEK293F cell, a Freestyle293F cell or an Expi293F cell;
optionally, the insect cell is a sf9 cell, a Hi5 cell, a sf21 cell or a S2 cell;
optionally, the yeast cell is a Pichia cerevisiae cell or a yeast cell transformed therefrom;
optionally, the bacterial cell is an Escherichia coli cell.

8. Use of the recombinant chimeric antigen according to any one of claims 1-5 for the preparation of a medicament for the prevention and/or treatment of poxvirus infection;
optionally, the poxvirus is selected from the group consisting of Monkeypox virus, Cowpox virus, Variola virus and Vaccinia virus;
optionally, the medicament is a vaccine, preferably a recombinant protein vaccine; further preferably, the recombinant protein vaccine comprises an adjuvant selected from the group consisting of an aluminum adjuvant, MF59 adjuvant and an MF59-like adjuvant;
optionally, the vaccine is in the form of a nasal spray, an oral preparation, a suppository or a parenteral preparation;
preferably, the nasal spray is selected from the group consisting of an aerosol, a spray and a powder aerosol;
preferably, the oral preparation is selected from the group consisting of a tablet, a powder, a pill, a granule, a soft/hard capsule, a film-coated preparation and a paste; further preferably, the tablet is a sublingual tablet; further preferably, the granule is a fine granule; further preferably, the powder is a bulk powder; further preferably, the pill is a minipellet;
preferably, the parenteral preparation is selected from the group consisting of a transdermal preparation, an ointment, a plaster, a topical liquid, an injectable preparation; further preferably, the injectable preparation is a bolus-injectable preparation.

9. A vaccine or immunogenic composition, comprising the recombinant chimeric antigen according to any one of claims 1-5, and a physiologically acceptable vehicle, adjuvant, excipient, carrier and/or diluent;
optionally, the vaccine or immunogenic composition is a poxvirus recombinant protein vaccine, which comprises the recombinant chimeric antigen according to any one of claims 1-5 and an adjuvant;
preferably, the adjuvant is one or more selected from the following adjuvants: an aluminum adjuvant, MF59 adjuvant and an MF59-like adjuvant.

10. The vaccine or immunogenic composition according to claim 9, **characterized in that** the vaccine or immunogenic composition is in the form of a nasal spray, an oral preparation, a suppository or a parenteral preparation;
preferably, the nasal spray is selected from the group consisting of an aerosol, a spray and a powder aerosols;
preferably, the oral preparation is selected from the group consisting of a tablet, a powder, a pill, a granule, a soft/hard capsule, a film-coated preparation and a paste;
further preferably, the tablet is a sublingual tablet;
further preferably, the granule is a fine granule;
further preferably, the powder is a bulk powder;
further preferably, the pill is a minipellet;
preferably, the parenteral preparation is selected from the group consisting of a transdermal preparation, an ointment, a plaster, a topical liquid, an injectable preparation; further preferably, the injectable preparation is a bolus-injectable preparation.
